# DEMANDE DE BREVET EUROPEEN

(11) **EP 2 238 999 A1**
(43) Date de publication de la demande: **13.10.2010**
(21) Numéro de dépôt: 09157227.1
(22) Date de dépôt: 02.04.2009
(51) Int. Cl.: A61M 5/20, A61M 5/24, A61M 5/32, A61M 5/315

(54) **Dispositif d'injection automatique**

(71) Demandeur: Pongpairochana, Vincent, 1093 La Conversion (CH)
(72) Inventeur: Pongpairochana, Vincent, 1093 La Conversion (CH)
(74) Mandataire: GLN

(57) **Abrégé**

La présente invention concerne un dispositif d'auto-injection (22) doté d'un compartiment articulé en référence au dispositif pour recevoir une seringue (10) munie d'une aiguille (18) protégée par un capuchon (20) de protection solidaire de la seringue, le compartiment étant susceptible d'évoluer entre une première position ouverte pour l'insertion et le retrait de la seringue, et une deuxième position fermée pour la réalisation de l'injection. Selon l'invention, le dispositif comporte un système de désolidarisation destiné à coopérer avec le capuchon de la seringue pour le désolidariser de la seringue, la désolidarisation étant conditionnée par le passage du compartiment à sa deuxième position. En outre, le passage du compartiment de la deuxième à la première position est conditionné par la solidarisation du capuchon.

## Description

### Domaine technique

La présente invention concerne un dispositif d'injection, doté d'un compartiment articulé en référence au dispositif pour recevoir une seringue munie d'une aiguille protégée par un capuchon de protection solidaire de la seringue. Le compartiment est susceptible d'évoluer entre une première position ouverte pour l'insertion et le retrait de la seringue, et une deuxième position fermée pour la réalisation de l'injection.

### Etat de la technique

De nombreux traitements nécessitent que la patient subisse des injections intramusculaires quotidiennes ou sous-cutanées fréquentes et à intervalles régulières, mensuels, hebdomadaires. Il est courant, pour permettre au patient de mener une vie normale, que celui-ci se fasse lui-même les injections. Dans ce but, plusieurs dispositifs ont été mis au point, avec notamment pour objectif de faciliter l'opération d'injection, tant du point de vue ergonomique que psychologique. Au sujet de ce dernier point, on peut aisément concevoir que la vision de l'aiguille peut faire peur au patient. De plus, le risque de blessure ou de contamination est crucial, notamment pour les personnes tierces devant manipuler les dispositifs.

Pour améliorer la sécurité des seringues de type classique, il a été développé des capuchons, passifs ou actifs, permettant de cacher l'aiguille. Néanmoins, ce genre de capuchons ne procure aucune amélioration de la fonctionnalité du dispositif d'injection et c'est toujours au patient, comme pour une seringue conventionnelle, d'effectuer l'action motrice pour réaliser l'injection.

Des dispositifs d'auto-injection proposent de suppléer le patient pour effectuer automatiquement l'injection, après le déclenchement de celle-ci. Il existe ainsi des dispositifs jetables. Leur coût est un inconvénient majeur dans le cas d'un traitement impliquant de fréquentes injections. En outre, étant donné que le dispositif est à usage unique, il est très peu perfectionné techniquement, notamment d'un point de vue contrôle, ou au niveau de moyens électroniques permettant de perfectionner les fonctionnalités.

Des dispositifs utilisent parfois des seringues conventionnelles, ce qui avantageux, car cela permet de combiner des éléments jetables pour les parties en contact avec le médicament, et des éléments pouvant être perfectionnés, car étant conservés d'une utilisation à une autre. Les protocoles de traitement sont généralement conçus pour que le contenu de la seringue soit injecté entièrement en une seule fois. Cependant, il est nécessaire d'effectuer beaucoup de manipulation, avant l'injection, pour retirer le capuchon protégeant la seringue, introduire la seringue dans le dispositif, puis, après l'injection, retirer la seringue du dispositif et remettre le capuchon. Ce genre de dispositifs présente l'inconvénient de devoir effectuer certaines de ces manipulations alors que l'aiguille n'est pas protégée. Les manipulations de la seringue usagée et contaminée après injection exposent le personnel hospitalier ou l'entourage du patient à des risques d'infection.

Un autre type de dispositif propose d'utiliser des cartouches multidoses de médicament, c'est-à-dire susceptibles d'être utilisées plusieurs fois consécutivement, l'appareil se chargeant d'administrer la bonne quantité au patient. Ce genre d'appareil peut être bien sécurisé pour le patient et les personnes devant l'avoir entre les mains, car l'aiguille d'injection est toujours cachée et ne présente pas de risque de blessure. Un tel appareil est particulièrement sophistiqué, notamment pour assurer de manière sûre le dosage du médicament, mais également pour assurer sa programmation, en fonction du traitement et du médicament. Ces appareils sont adaptés pour des cartouches multidoses, mais ne peuvent recevoir des seringues conventionnelles.

La présente invention a pour but de proposer un dispositif permettant de combiner les avantages des appareils ci-dessus, en évitant les inconvénients. Particulièrement, l'invention porte sur un dispositif très sûr, avantageux au niveau de l'impact sur le coût du traitement et pouvant être couplé à une électronique performante.

### Divulgation de l'invention

De manière plus précise, l'invention porte sur un dispositif d'auto-injection doté d'un compartiment articulé en référence au dispositif pour recevoir une seringue munie d'une aiguille protégée par un capuchon de protection solidaire de la seringue. Le compartiment est susceptible d'évoluer entre une première position ouverte pour l'insertion et le retrait de la seringue, et une deuxième position fermée pour la réalisation de l'injection. Selon l'invention et afin d'éviter tout risque de blessure ou de contamination avec l'aiguille, le dispositif comporte un système de désolidarisation destiné à coopérer avec le capuchon de la seringue pour le désolidariser de la seringue, la désolidarisation étant conditionnée par le passage du compartiment à sa deuxième position. En outre, le passage du compartiment de la deuxième à la première position est conditionné par la solidarisation du capuchon.

L'invention concerne également un système de gestion et de contrôle de protocoles d'injection, comprenant :
- un serveur stockant des données relatives au patient et à son traitement, et
- au moins un dispositif selon l'une des revendications précédentes pour réaliser les injections, comportant des moyens de connexion pour être relié à distance au serveur et des moyens électroniques,
**caractérisé en ce que** lesdits moyens électroniques sont agencés pour
- enregistrer l'historique des injections,
- suivre et contrôler le suivi du traitement et, éventuellement, rappeler au patient le moment des injections,
- transmettre au serveur ledit historique,
- recevoir et traiter des informations transmises par le serveur.

D'autres aspects de l'invention sont mentionnés dans les revendications de la présente demande.

### Brève description des dessins

D'autres caractéristiques de la présente invention apparaîtront plus clairement à la lecture de la description qui va suivre, faite en référence au dessin annexé, dans lequel:
- les figures 1 à 9 représentent différentes étapes successives de la mise en oeuvre d'un dispositif selon l'invention, les figures 1b et 2b étant des vues agrandies, et
- la figure 10 schématisant un système de gestion et de contrôle de protocoles d'injection.

### Mode(s) de réalisation de l'invention

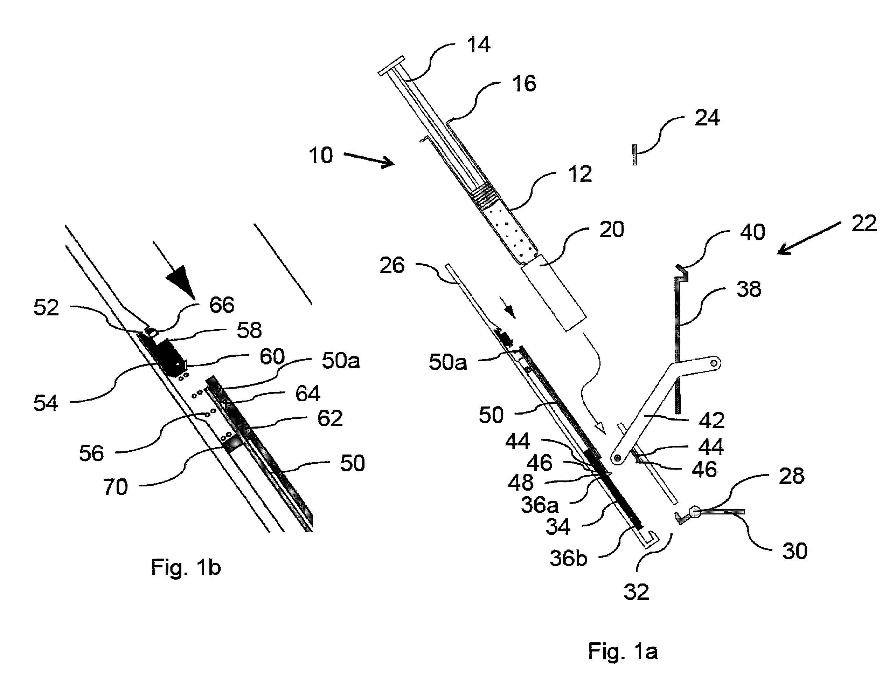

Les figures 1 à 9 représentent, de manière schématique, un dispositif permettant à un patient de s'auto-injecter le contenu d'une seringue 10 de type conventionnel. On entend par seringue conventionnelle, une seringue comprenant un corps 12 à l'intérieur duquel peut prendre place un liquide, pouvant être comprimé par un piston 14 mobile à l'intérieur du corps. En général, l'extrémité du corps 12 recevant le piston est muni d'une collerette 16, pour fournir un appui pour une injection manuelle. La seringue 10 peut recevoir, à son extrémité opposée à la collerette, une aiguille 18 creuse, pour injecter le liquide contenu dans le corps 12 de la seringue 10, dans le patient.

Pour protéger l'aiguille 18, celle-ci est recouverte, lorsque la seringue 10 n'est pas utilisée, d'un capuchon 20, en général réalisé en plastique rigide, solidarisé en force avec la seringue 10.

Sur les figures 1 à 9, le dispositif est représenté de manière schématique, afin de rendre plus claire la compréhension de l'invention. Ainsi, les éléments électroniques régissant l'injection, les moteurs et autres écrans pour l'affichage d'information, ne sont pas représentés sur les dessins. Pour ne pas surcharger les dessins, certains éléments n'ont été représentés que sur certaines figures.

Ainsi, la figure 1 montre un dispositif d'auto-injection 22 selon l'invention, dont une première paroi 24 comporte un volet 26 articulé sur une charnière 28. Cette dernière est, de préférence, située sur une deuxième paroi 30, adjacente à la première 24. On définit de la sorte un compartiment, articulé en référence au dispositif 22. Comme on va le comprendre par la suite, ce compartiment est destiné à recevoir une seringue 10, pour l'injection de son contenu.

La deuxième paroi 30 est percée, dans sa partie appartenant au compartiment, d'une ouverture 32 pour le passage de l'aiguille 18 de la seringue 10 et de son capuchon 20. Pour recevoir cette dernière, le compartiment comporte encore un anneau de guidage 34, solidaire du volet 26 et situé du côté de l'ouverture 32, de manière à guider la seringue 10 afin qu'elle soit dans la position souhaitée pour son insertion dans le compartiment et pour l'injection. L'anneau de guidage 34 présente, sur sa paroi intérieure, une première 36a et une deuxième 36b creusures, alignées selon l'axe longitudinal du compartiment et éloignées l'une de l'autre. Les creusures 36a et 36b sont identiques et forment une série. Plusieurs séries peuvent être réparties sur la circonférence de la paroi intérieure de l'anneau de guidage. Le rôle de ces creusures 36a et 36b apparaîtra plus loin dans la description.

Le compartiment comporte encore une cloison interne 38 située dans le dispositif, afin de parfaitement délimiter un logement pour la seringue 10. Cette cloison interne 38 présente un épaulement 40 pour coopérer avec la collerette 16 de la seringue 10. La cloison interne 38 est agencée de manière à prolonger l'anneau de guidage 34 lorsque le compartiment est dans une position fermée, c'est-à-dire lorsque le volet 26 est aligné sur la première paroi 24.

Un bras 42 est également monté pivotant à une première extrémité sur le dispositif. Une deuxième extrémité du bras 42 est montée pivotante sur le compartiment, pour tenir le volet 26 et définir une position d'ouverture maximale. Sont ainsi définies une première position d'ouverture maximale pour l'insertion et le retrait de la seringue, et une deuxième position fermée pour la réalisation de l'injection.

Selon un point intéressant de l'invention, le compartiment comprend des moyens de désolidarisation du capuchon en référence à la seringue. Ces moyens peuvent être formés par une bague 44 ajustée à l'intérieur de l'anneau de guidage 34. La deuxième extrémité du bras 42 est pivotée sur la bague 44, de sorte que l'ouverture et la fermeture du volet 26 en référence au dispositif, entraînent un mouvement de translation de la bague 44, respectivement en direction de l'ouverture 32 et en s'éloignant de l'ouverture 32.

Plus précisément, le diamètre extérieur de la bague 44 est légèrement inférieur au diamètre intérieur de l'anneau de guidage 34, de manière à ce que leur paroi, respectivement extérieure et intérieure, soient en contact l'une avec l'autre. La bague 44 comporte un ou plusieurs crochets 46, situés de préférence à l'extrémité de la bague 44. Les crochets 46 sont dotés d'un renflement 48 situé sur la paroi extérieure de la bague 44 et de forme complémentaire aux creusures 36a et 36b. Chaque crochet est destiné à coopérer avec les creusures d'une série. La figure 1a montre deux crochets 46, positionnés à des hauteurs différentes en référence au compartiment et, comme on le comprendra ci-après, destinés à coopérer différemment avec un capuchon 20.

La course de l'anneau de guidage 34 définie par la géométrie du bras 42, est égale à la distance séparant deux creusures 36a et 36b d'une série. Les crochets 46 sont positionnés de manière à ce que, lorsque le compartiment est dans sa première position d'ouverture maximale, les renflements 48 sont logés dans les premières creusures 36a et lorsque le compartiment est dans sa deuxième position fermée, les renflements 48 sont logés dans les deuxièmes creusures 36b. Naturellement, entre ces deux positions, les crochets 46 translatent entre les deux creusures 36a et 36b et les renflements 48 sont appuyés sur la paroi intérieure de l'anneau de guidage 34, ce qui contraint légèrement la bague de guidage 34 et rapproche les crochets 46 du centre de la bague de guidage 34. La forme des renflements 48 et les creusures 36 est adaptée pour ne pas avoir de blocage entre ces éléments.

On notera également que la bague de guidage 34 peut comporter un prolongement 50 situé du côté du volet, doté d'un doigt 50a dont le rôle apparaîtra plus loin.

Le dispositif d'injection 22 peut encore être doté d'un système de verrouillage du volet 26, non représenté. Ce système permet que le passage du compartiment de sa deuxième position de fermeture à une position d'ouverture, soit conditionné par la solidarisation du capuchon 20 en référence à la seringue 10. Ainsi, pour sécuriser l'utilisation du dispositif d'auto-injection et garantir qu'une seringue 10 ne peut être extraite du compartiment, particulièrement après une injection, que si le capuchon 20 de sécurité a été remis en place, un capteur, par exemple de type optique, peut être disposé dans le compartiment afin de détecter la présence du capuchon 20, à une hauteur signifiant qu'il est nécessairement solidarisé avec la seringue 10. Ce capteur peut être avantageusement relié à un loquet, non représenté, coopérant avec le volet 26 lorsque celui-ci est fermé. Le loquet peut être commandé électriquement de manière à être verrouillé et à ne pouvoir libérer le volet 26 que si le signal transmis pas le capteur indique que le capuchon 20 est bien en place sur la seringue 10. Naturellement, d'autres types de capteurs peuvent être utilisés pour détecter la présence du capuchon. Un système de verrouillage complètement mécanique pourrait également être envisagé.

De manière avantageuse, le dispositif d'auto-injection représenté au dessin comporte également un système de verrouillage de la seringue 10 dans le compartiment, qui permet, lorsque la seringue 10 a été complètement insérée dans le compartiment, de ne faire ressortir la seringue 10 que lorsque le volet 26 est complètement ouvert.

Dans ce but, la paroi intérieure du volet 26 comprend un ergot 52, formant butée pour un chariot 54, susceptible de coulisser le long de la paroi intérieure du volet 26. Un premier ressort 56 exerce une force sur le chariot 54 tendant à l'amener contre l'ergot 52. Le chariot 54 comporte encore un décrochement 58 agencé de manière à ce que la seringue, plus particulièrement sa collerette 16, puisse prendre appui sur lui, lorsqu'elle est insérée dans le compartiment. Le chariot 54 comporte encore un bec 60 monté rétractable, s'étendant en direction de l'intérieur du compartiment. L'intérieur du compartiment comporte encore une deuxième cloison interne 62, ajustée à l'intérieur de la bague 44 et comprenant sur sa paroi extérieure, une échancrure 64 de forme complémentaire au bec 60. Ce dernier est agencé de manière ce que le doigt 50a du prolongement 50 le fasse se rétracter lorsqu'ils se croisent dans une direction, en l'occurrence, lorsque le prolongement 50 se déplace en direction de l'extrémité du volet. Lorsque le prolongement 50 se déplace dans une autre direction, le bec est agencé de manière à ce que le doigt 50a puisse échapper du bec 60, sans qu'il y ait d'interaction entre ces éléments. Le bec 60 est positionné de manière à pouvoir coopérer avec l'échancrure 64 pour former des moyens de blocage.

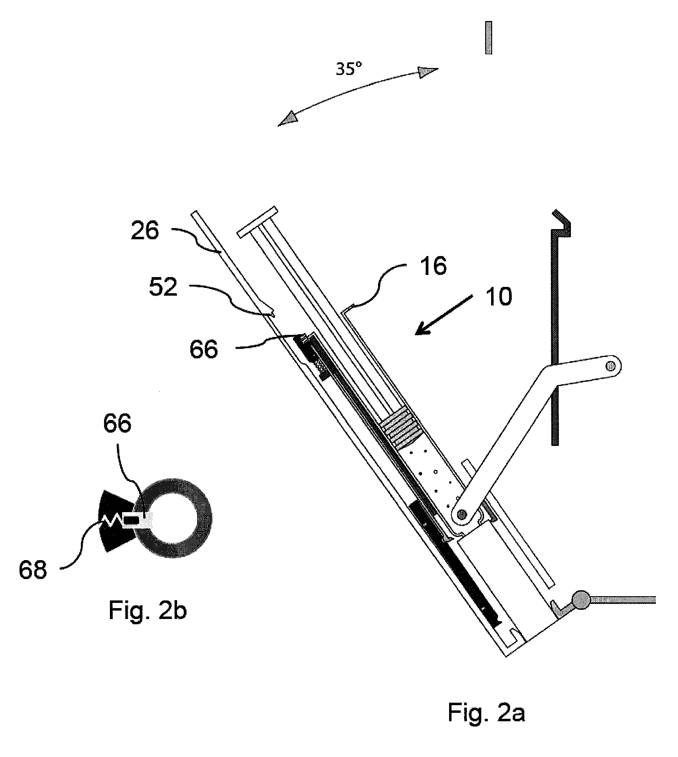

Le chariot 54 présente encore, au niveau du décrochement 58, une languette 66, mieux visibles sur la figure 2b. La languette 66 est mobile radialement et coopère avec un deuxième ressort 68, moins fort que le premier, exerçant une force tendant à l'entraîner vers le milieu du compartiment. L'ergot 52 est agencé de manière à, lorsque le chariot 54 est en butée contre lui, coopérer avec la languette 66 et contraindre le deuxième ressort 68 de manière à ce que la languette laisse libre le décrochement 58. Lorsque le chariot 54 n'est pas en butée contre l'ergot 52, le deuxième ressort amène la languette 66 au-dessus du décrochement 58. On notera que, dans ce dernier cas, un espace permettant de loger la collerette 16 subsiste entre le décrochement 58 et la languette 66.

La deuxième cloison interne 62 présente un renvoi 70 sur lequel prend appui le premier ressort 56. Ce renvoi 70 est percé d'une ouverture susceptible d'être traversée par le prolongement 50 de la bague 44.

Les différentes fonctions des éléments qui ont été décrits ci-dessus vont maintenant apparaître plus clairement à la lecture de la description qui suit, faite en référence aux figures 1 à 9.

La figure 1 montre le dispositif d'auto-injection selon l'invention, dont le volet 26 est ouvert, afin de permettre l'insertion d'une seringue d'injection 10. La position du bras 22 en référence au volet 26, fait que les renflements 48 des crochets 46 de la bague 44 sont positionnés dans les premières creusures 36a. Le prolongement 50 de la bague 44 positionne la deuxième cloison interne 62 vers l'extrémité du volet 26 et le premier ressort 56 pousse le chariot 54 en butée contre l'ergot 52.

Lors de l'insertion de la seringue 10 dans le compartiment, la collerette 16 de la seringue vient prendre appui sur le décrochement 58 du chariot 54 et l'utilisateur, en contraignant le premier ressort 56, amène ensuite le bec 60 se bloquer sur l'échancrure 64. Lorsque le volet 26 commence à être rabattu (figure 2), l'ergot 52 ne coopère plus avec la languette 66, celle-ci est pressée par le deuxième ressort 68 et recouvre la collerette 16, empêchant la seringue 10 de sortir accidentellement du compartiment. Il est nécessaire de ramener totalement le chariot 54 en butée contre l'ergot 52 manuellement ou en ouvrant complètement le volet 26 pour libérer la seringue, éventuellement en l'enlevant de force, mais en tout cas de manière volontaire.

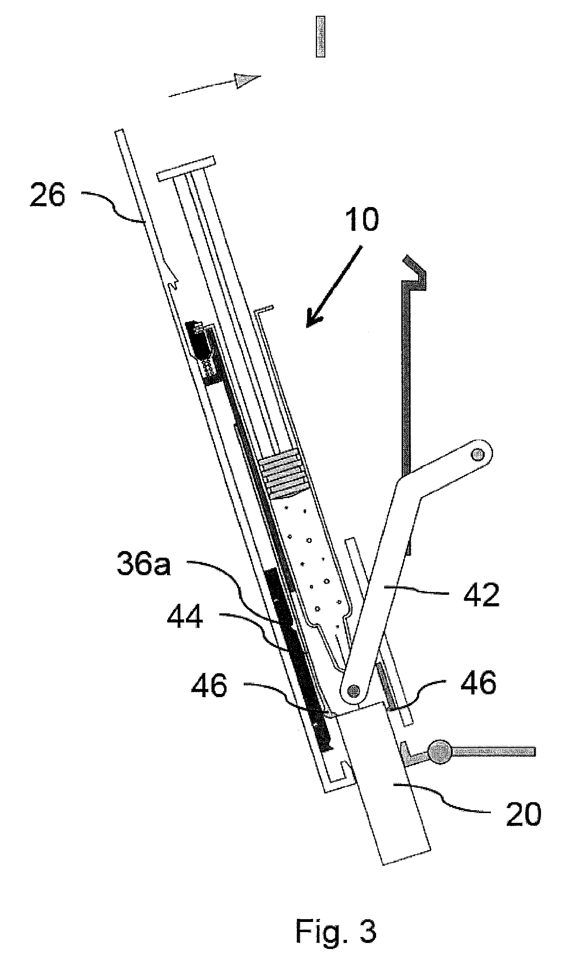

En poursuivant la fermeture du volet 26, le bras 42 pivote à ses deux extrémités et entraîne la bague 44 en translation. Les crochets 46 quittent les creusures 36a et se resserrent en direction de l'intérieur du compartiment de manière à venir coopérer avec le capuchon 20 de la seringue 10. En fonction de la configuration et de la disposition des crochets 46, cette coopération peut constituer en une poussée par le bord du capuchon 20. Les crochets peuvent également venir mordre dans le capuchon 20 de manière à le solidariser avec la bague 44. Avantageusement, on combine des crochets positionnés différemment, afin d'avoir une meilleure coopération entre eux et le capuchon 20 et de manière à pouvoir solidariser le capuchon sur la seringue en cas d'interruption de la fermeture. Ainsi, comme le montre la figure 3, la poursuite de la fermeture du volet permet de pousser le capuchon 20 et de le désolidariser de la seringue 10.

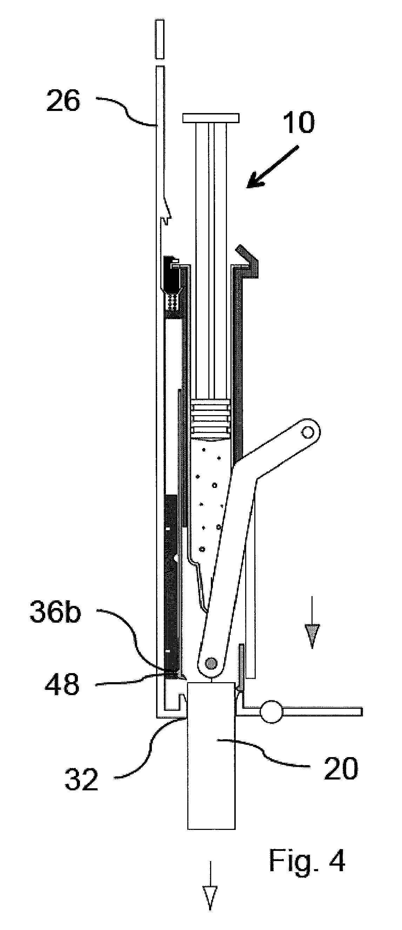

Lorsque le volet 26 est fermé (figure 4), les renflements 48 retombent dans les deuxièmes creusures 36b de manière à ne plus coopérer avec le capuchon 20. Celui-ci dépasse du dispositif par l'ouverture 32 qui peut avantageusement être dimensionnée de manière à légèrement retenir le capuchon 20, pour que celui-ci ne tombe pas. L'aiguille de la seringue reste en retrait de la surface extérieur du dispositif et l'ouverture suffisamment petite pour empêcher tout contact avec le patient. Le loquet du volet 26 est alors verrouillé, et il n'est pas possible de rouvrir le volet, tant que le capuchon n'est pas remis en place sur la seringue 10.

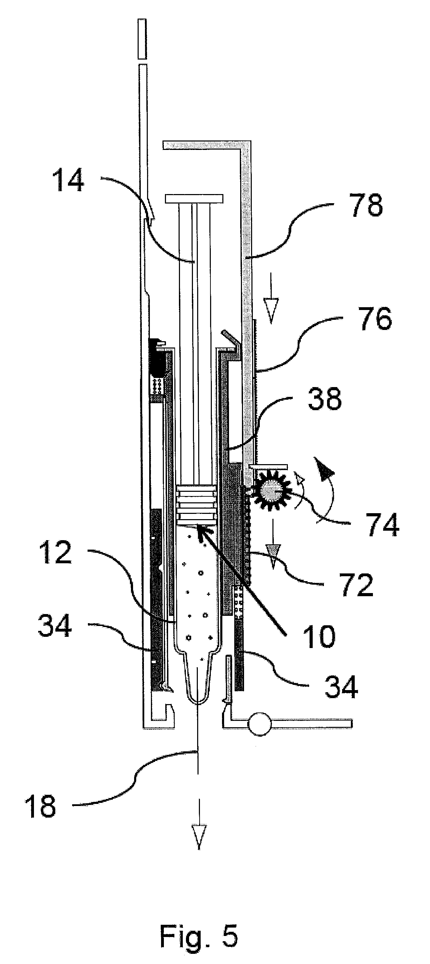

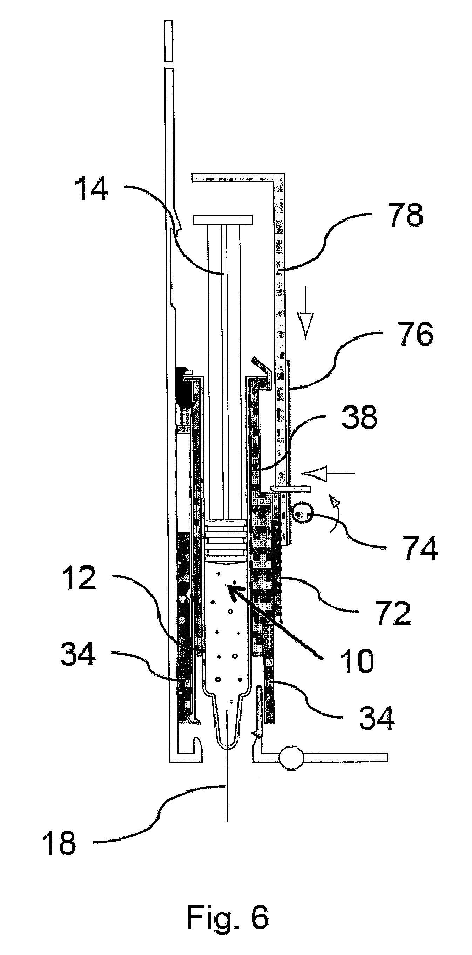

Le patient peut alors positionner le dispositif et déclencher l'injection (figures 5 et 6). La seringue 10 est déplacée dans le compartiment, en direction de l'ouverture 32, pour que l'aiguille 18 pénètre la peau du patient. Le piston 14 est actionné de manière à ce que tout ou partie du produit contenu dans le corps 12 soit injecté. Après l'injection, la seringue 10 est remontée à l'intérieur du dispositif et protégée de tout contact accidentel avec le patient ou une autre personne.

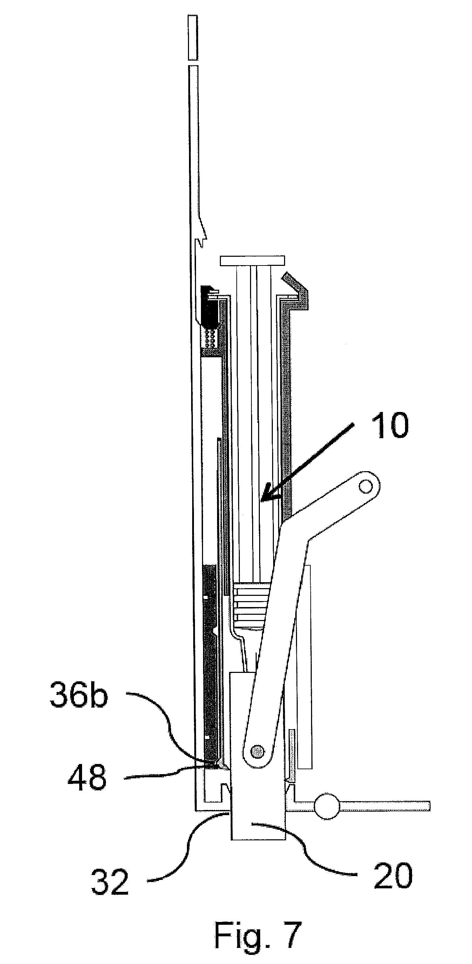

Les renflements 48 sont toujours dans les deuxièmes creusures 36b, de sorte que le patient peut librement insérer le capuchon 20 dans l'ouverture 32 et le solidariser sur la seringue 10, particulièrement après une injection. Le capteur détecte alors la présence du capuchon 20, ce qui déverrouille le loquet et autorise l'ouverture du volet (figure 7).

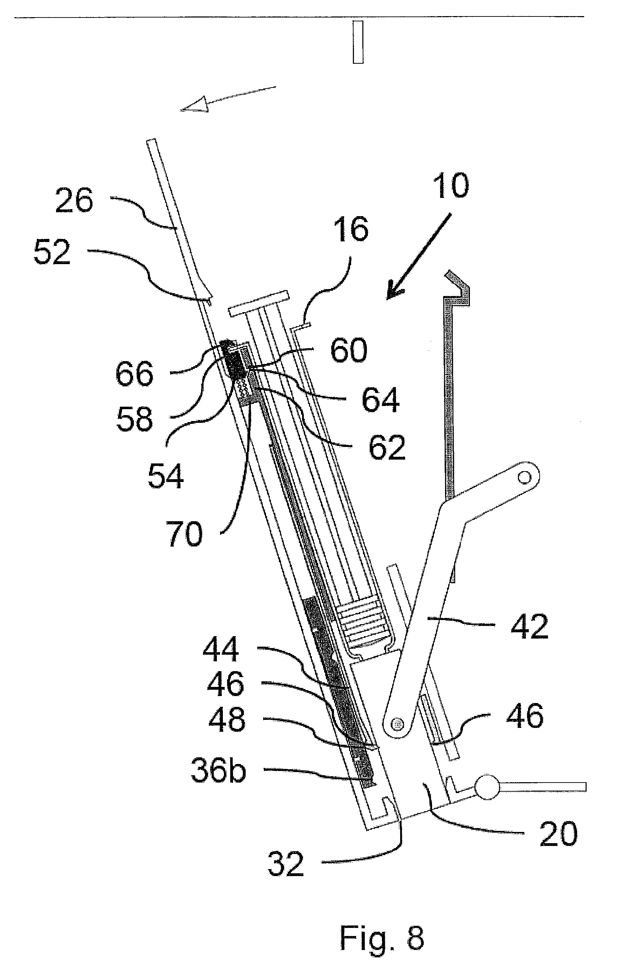

En ouvrant le volet (figure 8), typiquement par un bouton agissant sur le loquet, le mouvement du bras 42 ramène la bague 44 dans une direction opposée à l'ouverture 32. Ceci fait ressortir les renflements 48 de la deuxième creusure 36b et les crochets 46 viennent coopérer avec le capuchon 20, ce qui permet de garantir que celui-ci ne peut se désolidariser de la seringue 10, lors de l'extraction de celle-ci. On remarquera que, dans ce cas, tous les crochets 46 mordent dans le capuchon 20, car les renflements 48 sont situés dans les deuxièmes creusures 36b.

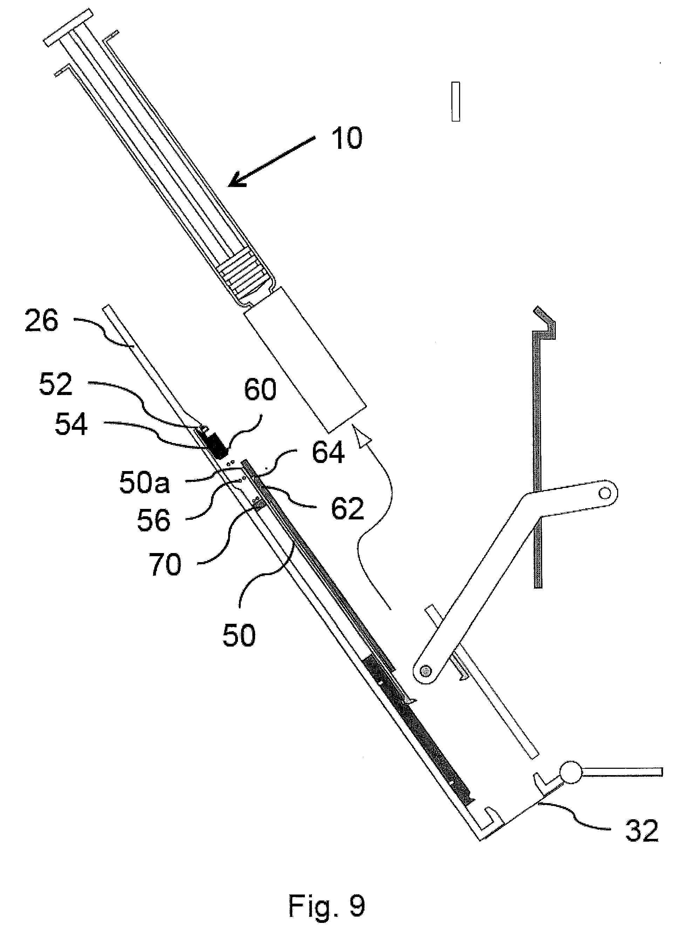

En poursuivant l'ouverture du volet 26, le doigt 50a du prolongement 50 traverse l'ouverture du renvoi 70 et écarte la deuxième cloison interne 62 de manière à libérer le bec 60 de l'échancrure 64. Le chariot 54 est alors librement soumis à l'action du premier ressort 56, qui le pousse en direction de l'ergot 52. Cela a pour effet d'amener la seringue 10 vers la sortie du compartiment, car la collerette 16 est encore prise entre le décrochement 58 et la languette 66. Enfin, lorsque le chariot 54 arrive en butée contre l'ergot 52, la languette 66 libère la collerette 16 et le patient peut sortir la seringue 10, protégée par son capuchon 20, en toute sécurité (figure 9).

Ainsi, le système de désolidarisation du capuchon 20 en référence à la seringue 10, coordonné avec la fermeture du volet 26, d'une part, et l'assujettissement du déverrouillage du loquet fermant le volet 26, d'autre part, permettent et garantissent que le patient insère et extraie la seringue avec son capuchon. Il est, de fait, impossible de se blesser avec l'aiguille, avant, pendant et après l'injection.

Le capteur détectant la présence du capuchon 20 peut être utilisé pour conditionner le déclenchement d'une injection au retrait de ce capuchon, de manière à ce qu'une injection ne puisse être déclenchée si le capuchon est toujours présent dans l'ouverture 32.

Pour la réalisation de l'injection, il est nécessaire, dans un premier temps, de déplacer toute la seringue en direction de l'ouverture, de manière à faire pénétrer l'aiguille dans la peau du patient. Dans un deuxième temps, il faut actionner le piston pour faire sortir le liquide contenu dans la seringue et l'injecter effectivement.

Pour la première étape, on peut voir, sur les figures 5 et 6, qu'une crémaillère 72 est montée solidairement à la cloison interne 38. Pour le passage de la crémaillère 72, une ouverture peut être ménagée dans l'anneau de guidage 34. Cette crémaillère coopère avec un premier niveau d'un pignon 74 entraîné par un moteur, non représenté.

Le pignon 74 comporte avantageusement un deuxième niveau, disposé de manière à coopérer avec une deuxième crémaillère 76 montée sur un pousse-piston 78. La première et la deuxième crémaillères sont agencées de manière à ce que, lors de la rotation du pignon 74, le premier puis le deuxième niveaux coopèrent successivement et respectivement avec les crémaillères 72 et 76. Ainsi sont successivement réalisées les première et deuxième étapes de l'injection. De manière avantageuse, un seul moteur est utilisé pour effectuer ces deux opérations. On peut éventuellement jouer sur les dentures des deux niveaux du pignon et des crémaillères ainsi que sur la vitesse de rotation du moteur pour adapter les vitesses d'entraînement.

Dans un deuxième mode de réalisation non représenté, les premières et les deuxièmes crémaillères peuvent être entraînées par deux moteurs différents. On relèvera également que le piston pourrait être entraîné directement par une vis sans fin coopérant avec le disque terminant généralement le piston, au lieu d'un entraînement par un pousse-seringue.

Le dispositif d'auto-injection selon l'invention est doté de moyens électroniques permettant de commander les différentes opérations effectuées au cours de l'injection, notamment la mise en fonction des moteurs pour la descente de la seringue et pour l'actionnement du piston. Les moyens électroniques sont également utilisés, comme mentionné ci-dessus, pour commander le verrouillage du loquet en fonction de la présence du capuchon sur la seringue.

Les moyens électroniques sont avantageusement programmables. La programmation peut se faire éventuellement par une interface directe, comportant un écran et des boutons de commande, ou par une interface indirecte, située à distance de l'appareil et communiquant avec le dispositif par Internet ou par liaison téléphonique.

Les moyens électroniques peuvent ainsi être programmés pour gérer des injections de doses, formées par des portions du contenu de la seringue. Dès lors que le dispositif est programmé de manière à connaître le produit présent dans la seringue et sa concentration, et la dose de médicament à administrer, le dispositif détermine la quantité de produit à injecter et, au moment de l'injection, l'administre. Le dispositif peut également intégrer un capteur électronique, par exemple optique, pour détecter la position du piston.

Les moyens électroniques peuvent, de manière avantageuse, être utilisés pour suivre et contrôler le bon suivi du traitement et, éventuellement, rappeler au patient le moment des injections. De préférence, les données relatives au patient et à son traitement son stockées à distance, sur un serveur avec lequel le dispositif communique.

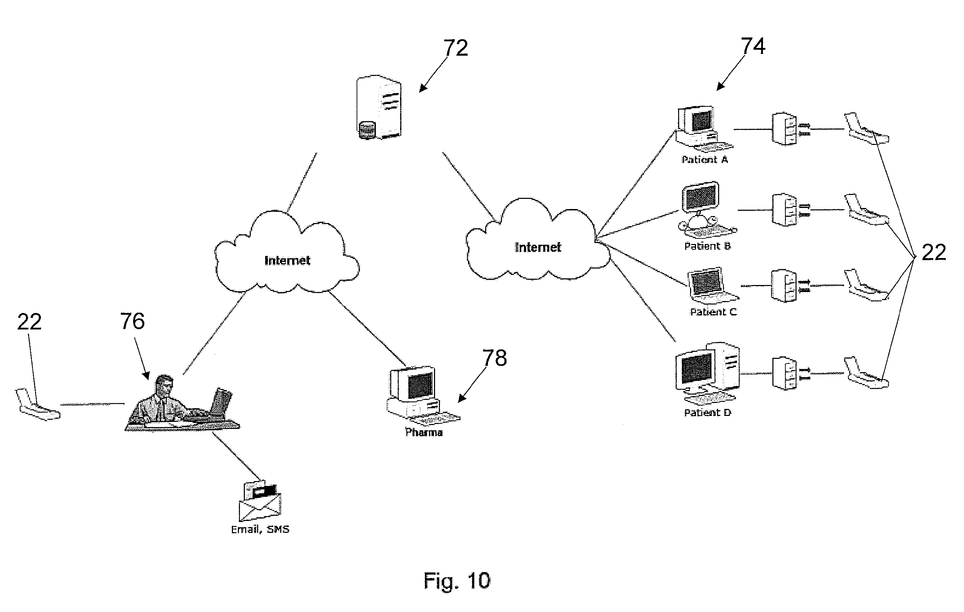

Ainsi, la figure 10 illustre un système de gestion et de contrôle de protocoles d'injection, comprenant :
- un serveur 72 stockant des données relatives au patient et à son traitement, et
- au moins un dispositif d'auto-injection 22 tel que décrit précédemment, comportant des moyens de connexion pour être relié à distance au serveur.

On a représenté sur la figure des dispositifs 22 pour des patients A, B, C et D.

Les moyens électroniques sont agencés pour :
- enregistrer l'historique des injections,
- suivre et contrôler le suivi du traitement et, éventuellement, rappeler au patient le moment des injections,
- transmettre au serveur ledit historique,
- recevoir et traiter des informations transmises par le serveur.

Dans des modes de réalisation préférés, les moyens électroniques peuvent, en outre, être programmés pour détecter et contrôler la nature et date limite d'utilisation de la seringue. En outre, ils peuvent aussi être utilisés pour réguler les paramètres d'injections de vitesse ou de temps. On peut encore relever que les moyens électroniques peuvent autoriser ou empêcher l'injection en fonction de la détection de l'absence de capuchon et de la présence de la peau.

Par exemple, on peut prévoir que le dispositif d'auto-injection se connecte au serveur par l'intermédiaire d'un téléphone portable, communicant sans fil avec le dispositif d'auto-injection. On peut également proposer que le dispositif doive être rechargé électriquement en étant branché à un ordinateur 74, par une connexion USB et que, à cette occasion, si l'ordinateur est connecté à Internet, le dispositif se relie automatiquement au serveur. Dans les deux cas, les moyens électroniques peuvent être programmés pour fournir au serveur un élément d'identification, qui permet de reconnaître le dispositif et le traitement associé au patient. Le serveur peut alors charger depuis le dispositif, l'historique des injections. Cet historique peut ensuite être consulté par le médecin 76, éventuellement lorsque celui-ci se connecte au serveur ou alors, en envoyant par email un fichier comportant l'historique. Le médecin peut, le cas échéant, modifier le programme d'injection du patient ou le dosage, ces modifications étant transmises au dispositif lors de sa prochaine connexion.

Les entreprises pharmaceutiques 78 peuvent également avoir la possibilité de se connecter au serveur 72, par exemple pour modifier des données relatives aux produits injectés ou prévenir de l'utilisation de certaines seringues identifiées par leur numéro de lot ou de série, notamment pour garantir qu'une seringue ne soit pas utilisée deux fois.

Les moyens électroniques peuvent être programmés pour fournir des signaux de rappel au patient, afin de l'avertir du moment auquel il doit effectuer la prochaine injection. Ces signaux peuvent être sonores, lumineux, ou consister en des messages affichés sur l'écran du dispositif. Les rappels peuvent aussi être envoyés au patient par le serveur, par email ou par SMS. En fonction des traitements et des maladies, le dispositif peut aussi envoyer un signal d'alarme lorsqu'une injection n'est pas faite ou après un certain délai pendant lequel le dispositif n'a pas été utilisé.

Naturellement, les différentes fonctions de communication du dispositif avec le serveur ou avec d'autres appareils peuvent être activées ou désactivées, soit par les fournisseurs de dispositif, soit par les médecins traitant les patients.

Ainsi est proposé un dispositif d'auto-injection perfectionné, destiné à être utilisé de manière répétée et permettant d'utiliser des seringues jetables, évitant que l'utilisateur doive manipuler la seringue avec son aiguille accessible. Le dispositif est également programmable, avantageusement à distance, pour un suivi aisé et efficace bilatéral, c'est-à-dire que tant le personnel soignant que le patient peut consulter, recevoir et transmettre des informations relatives au programme d'injections.

La description ci-dessus a été donnée à titre d'illustration non limitative de l'invention et l'homme du métier pourra prévoir d'autres modes de réalisation pour obtenir les fonctions décrites, sans sortir du cadre de l'invention revendiquée ci-dessous. On pourrait ainsi prévoir que le retrait du capuchon soit entièrement géré par les moyens électroniques, après la fermeture du volet.

## Revendications

1. Dispositif d'auto-injection (22) doté d'un compartiment articulé en référence au dispositif pour recevoir une seringue (10) munie d'une aiguille (18) protégée par un capuchon (20) de protection solidaire de la seringue, le compartiment étant susceptible d'évoluer entre une première position ouverte pour l'insertion et le retrait de la seringue, et une deuxième position fermée pour la réalisation de l'injection,
**caractérisé en ce que** le dispositif comporte un système de désolidarisation destiné à coopérer avec le capuchon de la seringue pour le désolidariser de la seringue, la désolidarisation étant conditionnée par le passage du compartiment à sa deuxième position,
et **en ce que** le passage du compartiment de la deuxième à la première position est conditionné par la solidarisation du capuchon.

2. Dispositif selon la revendication 1, **caractérisé en ce que** la désolidarisation est effectuée automatiquement lors du passage de la première à la deuxième position.

3. Dispositif selon la revendication 2, **caractérisé en ce que** le système de désolidarisation comporte des crochets (46) agencés pour coopérer avec le capuchon (20), lesdits crochets étant entraînés en translation en référence au capuchon, en fonction de la position du compartiment, entre une première et une deuxième positions, les crochets étant agencés de manière à ne pas coopérer avec le capuchon lorsqu'ils sont dans les première et deuxième positions, et à coopérer avec le capuchon lorsqu'ils sont entre les première et deuxième positions.

4. Dispositif selon l'une des revendications précédentes, **caractérisé en ce qu'**il est agencé de manière à laisser accessible le capuchon (20) après la désolidarisation, pour un retrait manuel, tandis que l'aiguille (18) demeure inaccessible à l'intérieur du dispositif.

5. Dispositif selon l'une des revendications précédentes, **caractérisé en ce qu'**il est agencé de manière à ce que, après une injection, l'aiguille est disposée à l'intérieur du dispositif de manière inaccessible, le capuchon pouvant être solidarisé à la seringue depuis l'extérieur.

6. Dispositif selon l'une des revendications précédentes, **caractérisé en ce qu'**il comporte un capteur agencé pour détecter la présence du capuchon solidarisé sur la seringue, et **en ce que** le capteur régit le verrouillage du compartiment dans sa deuxième position, le compartiment étant maintenu verrouillé tant que le capteur ne détecte pas la présence du capuchon.

7. Dispositif selon l'une des revendications précédentes, dans lequel est défini une position d'ouverture maximale, **caractérisé en ce qu'**il comporte un système de verrouillage de la seringue (10) dans le compartiment, agencé de manière à ce que, lorsque la seringue (10) a été complètement insérée dans le compartiment, la seringue (10) soit verrouillée dans le compartiment tant que ce dernier n'est pas revenu à sa position d'ouverture maximale.

8. Dispositif selon la revendication 6, **caractérisé en ce que** le système de verrouillage de la seringue coopère avec un organe ressort (56) agencé de manière à pousser la seringue (10) en direction de la sortie du compartiment lorsque le compartiment atteint sa position d'ouverture maximale.

9. Système de gestion et de contrôle de protocoles d'injection, comprenant :
- un serveur (72) stockant des données relatives au patient et à son traitement, et
- au moins un dispositif (22) selon l'une des revendications précédentes pour réaliser les injections, comportant des moyens de connexion pour être relié à distance au serveur et des moyens électroniques,
**caractérisé en ce que** lesdits moyens électroniques sont agencés pour
- enregistrer l'historique des injections,
- suivre et contrôler le suivi du traitement et, éventuellement, rappeler au patient le moment des injections,
- transmettre au serveur ledit historique,
- recevoir et traiter des informations transmises par le serveur.

10. Système selon la revendication 9, **caractérisé en ce que** lesdits moyens électroniques sont en outre agencés pour détecter et contrôler la nature et date limite d'utilisation de la seringue.

11. Système selon l'une des revendications 9 et 10, **caractérisé en ce que** lesdits moyens électroniques sont en outre agencés pour réguler les paramètres d'injections de vitesse ou de temps,

12. Système selon l'une des revendications 9 à 11, **caractérisé en ce que** les moyens de connexion sont agencés de manière à relier sans fil ledit dispositif à un téléphone portable, ledit téléphone portable reliant le dispositif audit serveur.
